# FASCICULE DE BREVET EUROPEEN

(11) **EP 1 480 569 B1**
(45) Date de publication et mention de la délivrance du brevet: **09.04.2008**
(21) Numéro de dépôt: 03739521.7
(22) Date de dépôt: 11.02.2003
(51) Int. Cl.: A61B 17/70

(54) **SYSTEME DE LIAISON ENTRE UNE TIGE RACHIDIENNE ET UNE BARRE TRANSVERSALE**
VERBINDUNGSSYSTEM ZWISCHEN EINER WIRBELSÄULENSTANGE UND EINEM QUERTRÄGER
CONNECTION SYSTEM BETWEEN A SPINAL ROD AND A TRANSVERSE BAR

(30) Priorité: 11.02.2002 FR 0201626
(43) Date de publication de la demande: 01.12.2004
(73) Titulaire: SCIENT'X, 78284 Guyancourt (FR)
(72) Inventeur: FRANCK, Bruno, Clinique du Lac et d'Argonay, F-74370 Argonay (FR); BEN MOKHTAR, Mourad, F-75018 PARIS (FR); RYAN, David, F-92210 SAINT CLOUD (FR)
(74) Mandataire: Thibault, Jean-Marc
(86) Numéro de dépôt international: PCT/FR2003/000425
(87) Numéro de publication internationale: WO 2003/068087

(56) Documents cités:
- FR-A- 2 736 535
- FR-A- 2 745 708
- FR-A- 2 771 918

## Description

La présente invention concerne le domaine technique de l'ostéosynthèse au sens général et elle vise en particulier les dispositifs assurant une liaison intervertébrale adaptée pour stabiliser le rachis ou pour corriger les déformations du rachis.

La présente invention vise plus précisément le domaine des systèmes adaptés pour assurer une liaison entre une tige d'ostéosynthèse rachidienne et une barre transversale.

Dans l'état de la technique, il est connu de nombreux dispositifs de liaison intervertébrale. D'une manière classique, un tel dispositif comporte des éléments d'ancrage osseux tels que par exemple, des vis d'implantation pédiculaire ou des crochets vertébraux équipés chacun d'une tête de fixation pour une tige de liaison reliant lesdits éléments entre eux. Les éléments d'ancrage osseux sont répartis le long de la zone du rachis à traiter pour permettre le montage de deux tiges de liaison s'étendant sensiblement parallèlement l'une à l'autre en étant disposées de chaque côté des apophyses épineuses des vertèbres. D'une manière avantageuse, au moins un entretoisement transversal entre les tiges de liaison est mis en oeuvre pour stabiliser et/ou obtenir un effet correcteur du montage réalisé (en distraction ou en compression).

Différents types de système de liaison entre une tige d'ostéosynthèse et une barre transversale ont été proposés dans l'art antérieur. Par exemple, le brevet FR 2 745 708 décrit un système de liaison constitué d'une première pièce en forme de mâchoire munie d'un passage pour la barre transversale et d'une vis de blocage susceptible de faire saillie dans le passage, et d'une seconde pièce en forme de mâchoire antagoniste articulée sur la première pièce. Le passage est adapté pour recevoir la barre de liaison transversale qui est susceptible, lors du serrage de la vis, de venir en appui contre les première et deuxième pièces pour assurer le rapprochement des mâchoires. Un tel système de liaison présente l'avantage de pouvoir être adapté directement sur des tiges d'ostéosynthèses rachidiennes, en n'importe quel endroit des tiges, en dehors bien entendu des sites d'implantation des vis pédiculaires et des crochets. De plus, les mâchoires de ce système entourent partiellement la tige d'ostéosynthèse, ce qui évite une intervention locale sur les vertèbres pour dégager l'espace nécessaire pour la mise en place dudit système de liaison.

Cependant, il doit être noté que l'opération de mise en place de ce système de liaison entre une barre transversale et une tige d'ostéosynthèse constitue une opération délicate à mener à bien. En effet, il est à considérer que le système de liaison doit être maintenu sur la tige d'ostéosynthèse tout en assurant le glissement de la barre transversale à travers le passage de réception. Le maintien du système doit être poursuivi pour permettre un léger serrage de la vis de blocage pour assurer le maintien en position du système de liaison tout en conservant une mobilité à l'ensemble pour la mise en place d'un autre système de liaison sur l'extrémité opposée de la barre transversale.

Pour tenter de remédier à ces inconvénients, le document FR 2 771 918 a proposé un connecteur pour un dispositif d'ostéosynthèse rachidienne réalisé sous la forme d'une pièce unique aménagée pour présenter deux mors s'étendant par des jonctions, à partir d'une tête de passage pour une barre transversale. Compte tenu de l'élasticité du matériau et des fentes réalisées au niveau des jonctions, les deux mors sont mobiles en rotation pour assurer le serrage de la tige d'ostéosynthèse. Un tel connecteur est ainsi clipsé sur la tige avant la mise en place de la barre transversale. Cependant, il s'avère que le connecteur est adapté sur la tige avec une force de serrage qui est difficilement maîtrisable compte tenu de la réalisation des mors. Il s'ensuit une difficulté pour la mise en place et le positionnement du connecteur sur la tige.

L'objet de l'invention vise donc à remédier aux inconvénients énoncés ci-dessus en proposant un système de liaison entre une tige d'ostéosynthèse rachidienne et une barre transversale, ne nécessitant pas une intervention préalable pour dégager la place nécessaire tout en offrant une simplicité de mise en place et une facilité pour l'exécution des opérations de liaison entre la barre transversale et la tige d'ostéosynthèse.

Pour atteindre un tel objectif le système de liaison entre une tige d'ostéosynthèse rachidienne et une barre transversale est aménagé pour présenter un passage pour la barre transversale destinée à exercer un effort sur deux mors montés articulés de manière à assurer leur rapprochement lors du serrage d'un organe de blocage. Selon l'invention, le système de liaison comporte un axe commun d'articulation sur lequel les mors sont articulés et un ressort de rappel monté pour exercer un appui sur les deux mors afin de les solliciter en rapprochement relatif indépendamment de la barre transversale de manière que les mors pincent la tige d'ostéosynthèse en vue d'assurer le montage stable du système de liaison sur ladite tige.

Selon une variante préférée de réalisation, le système de liaison comporte un moyen de limitation du rapprochement des mors sous l'action du ressort de rappel de manière qu'ils délimitent entre eux, en position de rapprochement maximale, un canal d'engagement pour la tige d'ostéosynthèse de sorte que lors de l'application d'un effort de poussée sur les mors en position d'appui sur la tige d'ostéosynthèse, les mors s'ouvrent pour enserrer et pincer ladite tige de sorte que le système de liaison est fixé sur ladite tige.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

La **figure 1** est une vue en perspective partielle montrant un dispositif de liaison rachidienne équipé de systèmes de liaison conformes à l'invention.

La **figure 2** est une vue en élévation d'un système de liaison conforme à l'invention.

La **figure 3** est une vue en perspective d'un système de liaison conforme à l'invention dont une partie a été arrachée.

La **figure 4** est une vue en élévation montrant le système de liaison entre une tige d'ostéosynthèse et une barre transversale.

La **figure 5** est une vue de dessus d'un système de liaison conforme à l'invention.

La **figure 6** est une vue en perspective d'une autre variante de réalisation d'un système de liaison conforme à l'invention.

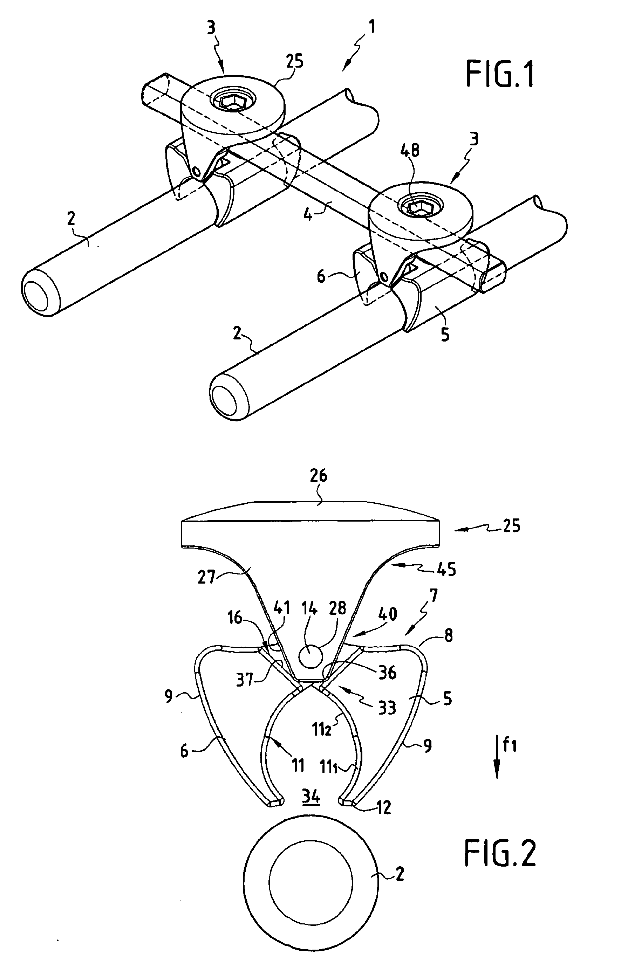

La **figure 1** représente de façon partielle un dispositif de liaison intervertébrale **1** comportant deux tiges **2** d'ostéosynthèses rachidiennes présentant chacune une section transversale circulaire et destinée à être montée sur des vertèbres à l'aide d'éléments d'ancrage osseux non représentés mais connu en soi, tel que des vis pédiculaires ou des crochets.

L'objet de l'invention concerne un système **3** de liaison entre la tige d'ostéosynthèse **2** et une barre transversale **4** qui possède, par exemple, une section droite transversale rectangulaire. Dans l'exemple illustré, la barre transversale **4** est destinée à assurer un entretoisement entre les deux tiges d'ostéosynthèses **2** de sorte que chaque extrémité de la barre transversale **4** est assemblée à une tige **2** par l'intermédiaire d'un système de liaison **3 c**onforme à l'invention.

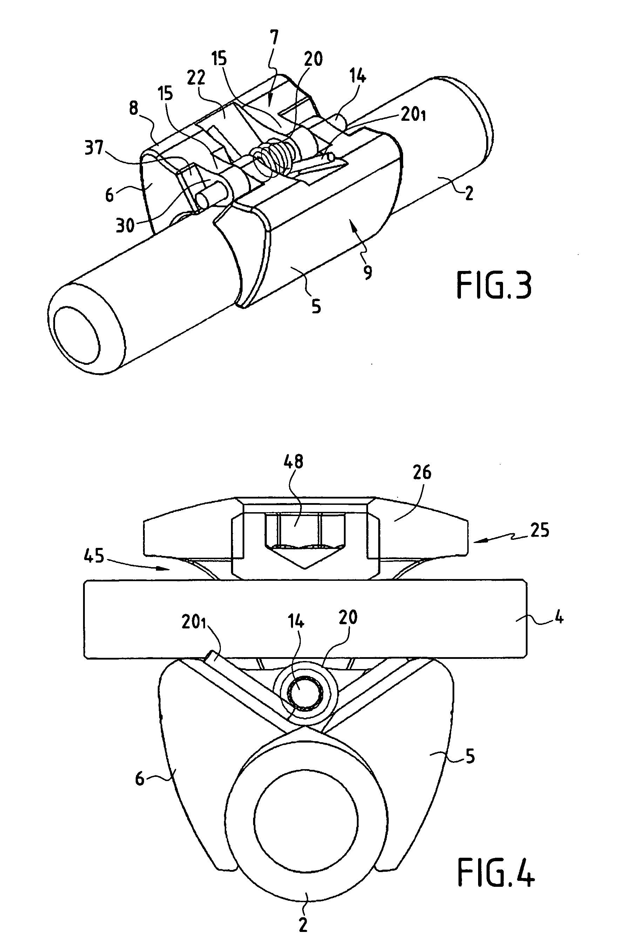

Le système de liaison **3** conforme à l'invention comporte un premier mors **5** et un deuxième mors **6** antagoniste au premier, pour former une pince ou un clip destiné à être monté sur une tige d'ostéosynthèse **2**. Tel que cela ressort plus précisément des **figures 2** à **4****,** chaque mors **5, 6** présente un profil général pouvant être assimilé à un triangle. Ainsi, chaque mors **5, 6** présente un plateau d'appui **7** prolongé vers l'extérieur, par l'intermédiaire d'un congé **8** de raccordement à une surface externe convexe **9**. Chaque mors **5, 6** comporte également une surface de serrage **11** de profil concave se raccordant à la surface externe **9** par un bord extrême **12.** Chaque mors **5, 6** possède ainsi un profil effilé limitant son encombrement.

Les mors **5, 6** qui sont réalisés par deux pièces distinctes sont montés articulés sur un axe commun d'articulation **14** en étant tournés l'un en face de l'autre. A cet effet, chaque mors **5, 6** est pourvu d'une paire d'oreilles **15** pourvues chacune d'un trou de passage pour l'axe commun d'articulation **14.** Les paires d'oreilles **15** sont aménagées au niveau d'une zone de liaison **16** entre la surface de serrage **11** et le plateau d'appui **7.** La paire d'oreilles **15** d'un mors s'étend de manière symétrique par rapport à la paire d'oreilles de l'autre mors. De plus, les paires d'oreilles **15** sont en saillie par rapport à la zone de liaison **16** des mors de manière à s'interpénétrer pour permettre l'alignement des trous de passage pour l'axe d'articulation **14.**

Selon une caractéristique de l'invention, le système de liaison 3 comporte au moins un ressort de rappel **20** sollicitant les mors **5, 6** en rapprochement relatif c'est à dire en fermeture de manière que les mors **5, 6** pincent la tige d'ostéosynthèse **2** en vue d'assurer le montage stable du système de liaison sur ladite tige **2.** Le ressort de rappel **20** est une pièce rapportée ou indépendante des mors 5, 6 et est monté pour exercer un appui sur les deux mors **5, 6** afin de les solliciter en rapprochement relatif. Dans l'exemple illustré, le ressort de rappel **20** est constitué par un ressort à boudins engagé sur l'axe d'articulation **14** et dont chacune de ses extrémités **20₁** vient en appui dans une échancrure **22** aménagée à partir des plateaux d'appui **7** des mors. Le ressort de rappel **20** se trouve ainsi logé entre les deux mors **5, 6** à l'intérieur de l'échancrure **22** aménagée entre les deux mors. Bien entendu, il pourrait être envisagé de réaliser le ressort de rappel **20** de manière différente par exemple sous la forme d'un ressort à lame.

Dans la mesure où les mors **5, 6** sont symétriques par rapport à l'axe d'articulation **14,** l'effort de serrage exercé sur la tige par les mors est symétriquement opposé en s'étendant sur deux surfaces opposées de cette tige. Il doit être compris que cette pince ou clip formé par les deux mors **5, 6** est apte à être monté sur des tiges possédant des diamètres de valeurs différentes tout en obtenant un serrage efficace sur la tige. Selon une variante préférée de réalisation, la surface de serrage **11** de chaque mors **5, 6** est constituée par des portées **11₁**, **11₂** de section différentes pour améliorer le contact surfacique avec la tige en fonction du diamètre des tiges **2.** Il est à noter que le dimensionnement des mors **5, 6** est adapté de manière qu'en position de serrage sur la tige **2,** leurs bords extrêmes **12** se trouvent toujours en retrait par rapport à un plan passant par la génératrice externe de la tige **2.** Les mors **5, 6** entourent ainsi partiellement la tige **2.**

Le système de liaison **3** comporte également un support **25** de montage des mors **5,6.** Tel que cela ressort plus précisément des **figures 1** et **2****,** le support **25** se présente sous la forme d'un étrier comportant une calotte **26** prolongée de part et d'autre, par des jambages **27** à chaque extrémité desquels est aménagé un alésage **28** de réception d'une extrémité de l'axe d'articulation **14.** Les jambages **27** et les mors **5, 6** sont aménagés pour autoriser les mouvements des mors en vue de leur encliquetage sur la tige **2.** A cet effet, chaque mors **5, 6** comportent au niveau de sa zone de liaison **16,** un dégagement **30** aménagé à partir du plateau d'appui **7** en s'ouvrant sur les faces latérales des mors.

Selon une caractéristique préférée de réalisation, le système de liaison **3** comporte un moyen **33** de limitation du rapprochement des mors **5, 6** sous l'action du ressort de rappel **20** de manière qu'ils délimitent entre eux, en position de rapprochement maximal illustrée à la **figure 2****,** un canal d'engagement **34** pour la tige d'ostéosynthèse **2** de sorte que lors de l'application d'un effort de poussée sur les mors en position d'appui sur la tige **2,** les mors s'ouvrent pour enserrer et pincer ladite tige. Dans cette position de rapprochement maximal, les bords extrêmes **12** des mors se trouvent écartés l'un de l'autre pour permettre l'ouverture progressive des mors permettant l'engagement de la tige **2** entre les mors **5, 6** pour venir la pincer de sorte que le système de liaison **3** est fixé sur ladite tige.

Dans l'exemple de réalisation illustrée, le moyen de limitation de rapprochement **33** est constitué par des butées **36** réalisées par le bord des jambages **27** sur lequel vient en appui une paroi de fond **37** délimitant le dégagement 30. Il doit être compris que les mors **5, 6** en position de repos sont sollicités par le ressort de rappel **20** de manière à occuper leur position maximale de rapprochement correspondant à la mise en appui des mors **5, 6** sur les butées **36.**

Selon une autre caractéristique préférée de réalisation, le système de liaison **3** comporte un moyen **40** de limitation de l'écartement des mors **5, 6** sous l'action d'un effort d'écartement notamment intempestif des mors **5, 6** en vue d'éviter l'endommagement du ressort de rappel **20.** Ce moyen de limitation d'écartement **40** est constitué par le rebord **41** des jambages **27** sur lequel peut venir en contact la paroi de fond **37** des mors.

Le système de liaison **3** présente également un passage **45** pour la barre transversale **4.** Dans l'exemple de réalisation illustré sur les dessins, le passage **45** est délimité en partie par le support **25** c'est à dire par les faces internes des jambages **27** et de la calotte **26** et par le plateau d'appui **7** des mors. Ce passage **45** possède une section rectangulaire orientée selon un axe sensiblement orthogonal à l'axe d'articulation **14** des mors **5, 6.**

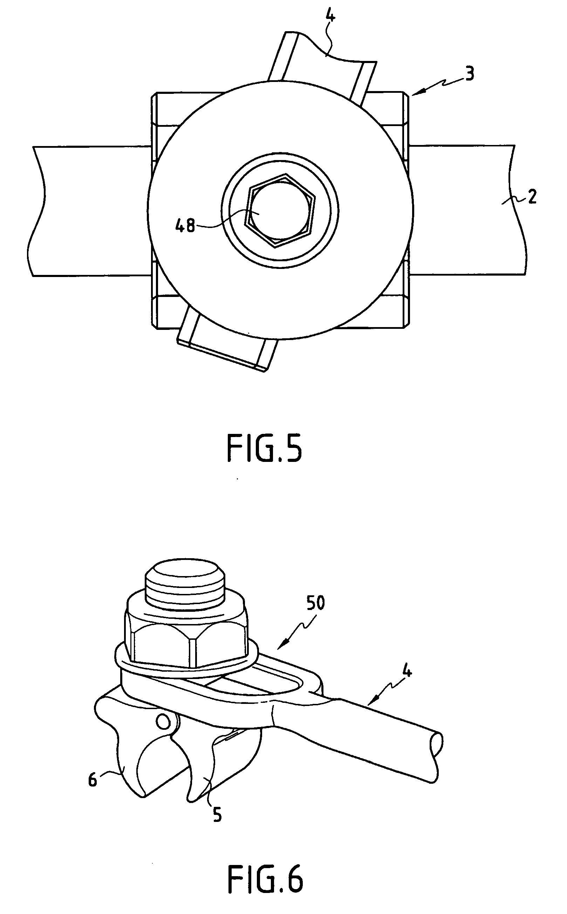

Le système de liaison **3** comporte également un organe de serrage **48** tel qu'une vis destinée à faire saillie à l'intérieur du passage **45.** Cette vis de blocage **48** est vissée dans un taraudage aménagé sur la calotte **26** de manière à être accessible à partir de la face externe de la calotte **26 (****figure 5****).**

La mise en oeuvre d'un système de liaison 3 entre une tige d'ostéosynthèse **2** et une barre transversale **4** découle directement de la description qui précède.

Après la mise en place d'une tige d'ostéosynthèse rachidienne **2** selon les techniques connues, il peut être procédé au montage stable du système de liaison **3** sur ladite tige **2** à l'endroit souhaité. A cet égard, le système de liaison **3** généralement dépourvu de la barre transversale **4,** est déplacé, à l'aide d'un ancillaire adapté, selon une direction sensiblement perpendiculaire à l'axe de la tige en plaçant les bords extrêmes **12** des mors orientés vers la tige d'ostéosynthèse **2.** Tel que cela apparaît plus précisément à la **figure 2****,** le système de liaison **3** est déplacé selon le sens de la flèche **f₁**. Lors de ce déplacement, les mors **5, 6** viennent en appui sur la tige **2** de sorte que la poursuite de l'effort de poussée selon le sens **f₁** conduit à l'ouverture progressive des mors qui viennent enserrer et pincer ladite tige. Il est à noter que l'ouverture des mors **5, 6** s'effectue automatiquement et facilement compte tenu de la présence du canal d'engagement **34** délimité entre les bords extrêmes **12** des mors. Après la suppression de l'effort de poussée, les mors **5, 6** exercent, sous l'action du ressort de rappel **20,** un effort suffisant de pincement autour de la tige **2** pour permettre son montage stable sur la tige **2** sans aucune intervention extérieure.

Le positionnement stable d'un tel système de liaison **3** sur la tige **2** facilite l'opération ultérieure d'insertion de la barre transversale **4** à l'intérieur du passage **45.** Bien entendu, dans le cas où une barre transversale **4** est placée entre deux tiges d'ostéosynthèse **2,** une opération identique de montage d'un système de liaison **3** conforme à l'invention est effectué sur l'autre tige d'ostéosynthèse. Tel que cela apparaît plus précisément à la **figure 5****,** il est à noter que la largeur du passage **45** est supérieure à la largeur de la barre **4** pour permettre un débattement angulaire de la barre par rapport à la tige **2.**

Après le positionnement de la barre transversale **4,** la vis de blocage **48** est vissée de manière que la barre **4** assure un appui sur les mors **5, 6** en vue d'assurer leur rapprochement pour permettre le blocage de la tige **2** par les mors **5, 6.** A cet effet, les congés de raccordement **8** des plateaux d'appui s'étendent à un niveau supérieur par rapport à l'axe d'articulation **14** permettant l'appui de la barre sur ces zones et par suite, le pivotement des mors **5** et **6** selon un sens de rapprochement.

Dans l'exemple illustré aux **figures 1** à **5****,** la barre transversale **4** exerce directement un effort sur les mors **5, 6** sous l'action de la vis de serrage **48.** La **figure 6** illustre un autre exemple de réalisation dans lequel la barre **4** est pourvue d'une boutonnière **50** en contact sur les plateaux d'appui **7** des mors et sur lequel agit un organe de blocage **48** constitué par un écrou emmanché sur une tige filetée portée par le support **25.** Il est à noter qu'il peut être envisagé que l'effort transmis par la barre **4** aux mors **5, 6** soit exercé indirectement par l'interposition d'une rondelle entre les mors et la barre 4.

L'invention n'est pas limitée aux exemples décrits et représentés car diverses modifications peuvent y être apportées sans sortir de son cadre.

## Revendications

1. - Système de liaison entre une tige d'ostéosynthèse rachidienne **(2)** et une barre transversale **(4),** le système étant aménagé pour présenter un passage **(45)** pour la barre transversale **(4)** destinée à exercer un effort sur deux mors **(5, 6)** montés articulés de manière à assurer leur rapprochement lors du serrage d'un organe de blocage **(48), caractérisé en ce qu'**il comporte :
- un axe commun d'articulation **(14)** sur lequel sont articulés les deux mors **(5, 6),**
- un ressort de rappel **(20)** monté pour exercer un appui sur les deux mors **(5, 6)** afin de les solliciter en rapprochement relatif indépendamment de la barre transversale, de manière que les mors **(5, 6)** pincent la tige d'ostéosynthèse en vue d'assurer le montage stable du système de liaison sur ladite tige.

2. **-** Système de liaison selon la revendication **1**, **caractérisé en ce qu'**il comporte un moyen **(33)** de limitation du rapprochement des mors **(5, 6)** sous l'action du ressort de rappel **(20)** de manière qu'ils délimitent entre eux, en position de rapprochement maximale, un canal d'engagement **(34)** pour la tige d'ostéosynthèse **(2)** de sorte que lors de l'application d'un effort de poussée sur les mors en position d'appui sur la tige d'ostéosynthèse **(2),** les mors **(5, 6)** s'ouvrent pour enserrer et pincer ladite tige de sorte que le système de liaison est fixé sur ladite tige.

3. **-** Système de liaison selon la revendications 1 ou 2, **caractérisé en ce que** le ressort de rappel **(20)** est engagé sur l'axe d'articulation **(14)** en étant logé dans une échancrure **(22)** aménagée dans les mors.

4. **-** Système de liaison selon l'une des revendications 1 à 3, **caractérisé en ce que** les mors **(5, 6)** sont symétriques en présentant chacun une surface de serrage **(11)** agissant de façon antagoniste sur la tige d'ostéosynthèse **(2)** et permettant leur montage sur des tiges de diamètres de valeurs différentes.

5. **-** Système de liaison selon la revendication 4, **caractérisé en ce que** les mors **(5, 6)** comportent chacun une surface de serrage **(11)** constituée par des portées **(11₁, 11₂)** de sections différentes.

6. - Système de liaison selon la revendication 3, **caractérisé en ce que** l'axe d'articulation **(14)** est porté par un support **(25)** délimitant le passage **(45)** pour la barre transversale **(4),** le support **(25)** étant pourvu de l'organe de blocage **(48)** destiné à faire saillie à l'intérieur du passage **(45).**

7. - Système de liaison selon la revendication 6, **caractérisé en ce que** le passage **(45)** s'étend selon une direction sensiblement perpendiculaire à l'axe d'articulation **(14)** des mors, selon une largeur supérieure à la barre transversale **(4)** pour autoriser un débattement angulaire à ladite barre.

8. **-** Système de liaison selon l'une des revendications 1 à 7, **caractérisé en ce qu'**il comporte des butées **(40)** pour limiter l'écartement entre les mors.

## Claims

1. A connection system between a spinal osteosynthesis rod (2) and a transverse bar (4), the system being arranged to present a passage (45) for the transverse bar (4) intended to exert a force on two clamping jaws (5, 6) mounted articulated so as to ensure that they move together when the locking element (48) is tightened, **characterised in that** it comprises:
- a common axis of articulation (14) on which are articulated the two clamping jaws (5, 6),
- a return spring (20) mounted to act on the two clamping jaws (5, 6) to move them towards one another relatively independently of the transverse bar, such that the clamping jaws (5, 6) grip the osteosynthesis rod and ensure that the connection system is firmly mounted on said rod.

2. The connection system as claimed in Claim 1, **characterised in that** it comprises means (33) for limiting the coming together of the clamping jaws (5, 6) under the action of the return spring (20), such that they delimit between them, in the maximum coming together position, an engagement channel (34) for the osteosynthesis rod (2) such that during application of a thrust force on the clamping jaws in a support position on the osteosynthesis rod (2), the clamping jaws (5, 6) open to enclose and grip said rod so that the connection system is fixed on said rod.

3. The connection system as claimed in Claims 1 or 2, **characterised in that** the return spring (20) is engaged on the articulation axis (14) by being lodged in a notch (22) arranged in the clamping jaws.

4. The connection system as claimed in any one of Claims 1 to 3, **characterised in that** the clamping jaws (5, 6) are symmetrical, each presenting a gripping surface (11) acting antagonistically on the osteosynthesis rod (2) and enabling them to be mounted on rods having different diameters.

5. The connection system as claimed in Claim 4, **characterised in that** the clamping jaws (5, 6) each comprise a gripping surface (11) constituted by spans (11₁, 11₂) of different cross-sections.

6. The connection system as claimed in Claim 3, **characterised in that** the articulation axis (14) is carried by a support (25) delimiting the passage (45) for the transverse bar (4), the support (25) being provided with a locking element (48) to project inside the passage (45).

7. The connection system as claimed in Claim 6, **characterised in that** the passage (45) extends according to a direction substantially perpendicular to the articulation axis (14) of the clamping jaws, according to a width greater than the transverse bar (4) to enable clearance angular to said bar.

8. The connection system as claimed in any one of Claims 1 to 7, **characterised in that** it comprises stops (40) for limiting the spread between the clamping jaws.

## Patentansprüche

1. System zur Verbindung einer Radikosteosynthesestange (2) mit einem Querstab (4), wobei das System derart vorgesehen ist, daß es einen Durchgang (45) für den Querstab (4) aufweist, der dazu bestimmt ist, auf zwei Spannbacken (5, 6), die gelenkig montiert sind, um ihre Annäherung bei Festklemmen eines Feststellelements (48) zu gewährleisten, eine Kraft auszuüben, **dadurch gekennzeichnet, daß** es umfaßt:
- eine gemeinsame Gelenkachse (14), auf der die beiden Spannbacken (5, 6) angelenkt sind,
- eine Rückstellfeder (20), die derart montiert ist, daß sie einen Druck auf die beiden Spannbacken (5, 6) ausübt, um sie unabhängig von dem Querstab einander relativ anzunähern, so daß die Spannbacken (5, 6) die Osteosynthesestange einklemmen, um die stabile Montage des Verbindungssystems auf der Stange zu gewährleisten.

2. Verbindungssystem nach Anspruch 1, **dadurch gekennzeichnet, daß** es ein Mittel (33) zur Begrenzung der Annäherung der Spannbacken (5, 6) unter der Wirkung der Rückstellfeder (20) umfaßt, so daß sie zwischen sich in der maximalen Annäherungsposition einen Eingreifkanal (34) für die Osteosynthesestange (2) definieren, so daß sich beim Anlegen einer Schubkraft an die Spannbacken in der Abstützungsposition auf der Osteosynthesestange (2) die Spannbacken (5, 6) öffnen, um die Stange zu umgeben und einzuklemmen, so daß das Verbindungssystem auf der Stange befestigt wird.

3. Verbindungssystem nach Anspruch 1 oder 2, **dadurch gekennzeichnet, daß** die Rückstellfeder (20) auf der Gelenkachse (14) im Eingriff steht, wobei sie sich in einem bogenförmigen Ausschnitt (22) befindet, der in den Spannbacken vorgesehen ist.

4. Verbindungssystem nach einem der Ansprüche 1 bis 3, **dadurch gekennzeichnet, daß** die Spannbacken (5, 6) symmetrisch sind, wobei sie jeweils eine Klemmfläche (11) aufweisen, die auf antagonistische Weise auf die Osteosynthesestange (2) einwirkt und ihre Montage auf Stangen mit Durchmessern von unterschiedlichen Werten ermöglicht.

5. Verbindungssystem nach Anspruch 4, **dadurch gekennzeichnet, daß** die Spannbacken (5, 6) jeweils eine Klemmfläche (11) aufweisen, die von Bereichen (11₁, 11₂) mit unterschiedlichen Querschnitten gebildet ist.

6. Verbindungssystem nach Anspruch 3, **dadurch gekennzeichnet, daß** die Gelenkachse (14) von einer Stütze (25) getragen wird, die den Durchgang (45) für den Querstab (4) begrenzt, wobei die Stütze (25) mit dem Feststellelement (48) versehen ist, das dazu bestimmt ist, in das Innere des Durchgangs (45) zu ragen.

7. Verbindungssystem nach Anspruch 6, **dadurch gekennzeichnet, daß** sich der Durchgang (45) in eine im wesentlichen auf die Gelenkachse (14) der Spannbacken senkrechte Richtung entlang einer größeren Breite als der Querstab (4) erstreckt, um einen Winkelausschlag des Stabs zu gestatten.

8. Verbindungssystem nach einem der Ansprüche 1 bis 7, **dadurch gekennzeichnet, daß** es Anschläge (40) umfaßt, um die Entfernung der Spannbacken zu begrenzen.
